# EUROPEAN PATENT APPLICATION

(11) **EP 1 496 038 A1**
(43) Date of publication of application: **12.01.2005**
(21) Application number: 03015576.6
(22) Date of filing: 11.07.2003
(51) Int. Cl.: C07B 61/00, G01N 33/53

(54) **A method for the generation of a dynamic combinatorial library using nitrones**

(71) Applicant: Therascope AG, 69120 Heidelberg (DE)
(72) Inventor: Hochgürtel, Matthias, 69198 Schriesheim (DE)
(74) Representative: Huhn, Michael, Dr.

(57) **Abstract**

The present invention relates to a method of forming a dynamic combinatorial library of compounds which are potentially capable of binding to a target, which method comprises
i) selecting one first set of molecules which molecules each carry at least one hydroxylamine function;
ii) selecting at least one further set of molecules which molecules carry at least one carbonyl function;
iii) reacting the two sets of molecules with each other and at least temporarily in the presence of the target, under conditions where a reversible formation of nitrone functions between the hydroxylamine function and the carbonyl function on the molecules forming each of the sets occurs.

Pharmaceutically active compounds which are formed when the library is generated can be identified by analysing the library, preferably by comparing the amounts of the compounds formed in the presence of the target and in the absence of the target.

## Description

The present invention relates to dynamic combinatorial chemistry (DCC), more precisely to a process for the generation of a dynamic combinatorial library (DCL) wherein nitrones are used.

Biologically active compounds and medical drugs were traditionally generated by conventional chemical or biological methods by variation of so-called lead compounds. Since the generation of these compound variants is time-consuming, only a relatively small pool (a small library) of these compound variants is accessible for biological tests.

These problems can be overcome by combinatorial chemistry (CC), a method where a set consisting of various basis components is used to generate a variety of new compounds by successive and repetitive application of specific chemical reactions. The covalent nonreversible connections between the components are performed individually in parallel or concertedly in the same compartment(s). So, vast combinatorial libraries (CLs) of extensive collections of constituents are available in a very short time. CC is therefore a very powerful method for exploring the molecular geometrical and interactional spaces through molecular diversity generation and is thus based on large populations of different molecules that are present as discrete entities.

In contrast to this, the so-called dynamic combinatorial chemistry (DCC) is a conceptually different approach that requires a reversible assembly process and its constituents may be either molecular or supramolecular.

DCC thus relies on reversible reactions or interactions between sets of basic components to give access to dynamic combinatorial libraries (DCLs) of potential entities, allowing the target-driven generation or amplification of the active constituents of the libraries. It thus represents a self-screening process by which the desired optimal species are preferentially expressed and retrieved from the DCL. DCC extends beyond static combinatorial chemistry (SCC) towards adaptive/evolutive chemical systems. It is especially useful for the search of ligands which bind to targets like enzymes, receptors or antibodies. Suitable detected ligands may be used as medicaments.

Several functional groups lend themselves for use in the generation of a DCL. Appropriate groups include aldehyde, keto, thiol, amino and hydrazine groups. The resulting molecules - which have been formed from at least two molecules present in the library - are linked by a unit formed in the reaction between the functional groups in the starting molecules. Examples are imines, hydrazones and disulfides.

DCLs can be generated in aqueous media as well as in organic solvents. They can be used for the generation and identification of ligands (in the presence of a given receptor) as well as for receptors (in the presence of a given ligand). For the identification of pharmacologically active compounds (which have an activity against a receptor, e.g. an enzyme), the DCL has to be generated in an aqueous medium, with the pH preferably being close to neutrality, to mimic physiological conditions. Under non-physiological conditions or even in non-aqueous, organic media, the identification of active compounds may be hampered, due to the lack of stability of the target, or the compounds identified are not active *in vivo*.

When designing a DCL, two different procedures were employed hitherto. In one procedure a), library generating and screening is performed in one single step; in the other procedure b), these two steps are carried out separately. The particulars for the above-mentioned different procedures are the following:
a) adaptive, combinatorial libraries (one-step procedure): the generation of the library constituents is conducted in a single step in the presence of the target, so that the library composition may adjust leading to selection and amplification of the preferred substrate(s); screening by the target occurs in parallel with the reversible generation of the library constituents; this is the approach where the dynamic features are operative over the whole process;
b) pre-equilibrated dynamic combinatorial libraries (pDCL) (two-step procedure): the constituents of the library are generated by reversible interconversion and equilibration in absence of the target, which is added in a second step after reversibility has been stopped; this has the advantage that one may use reversible reactions which are not compatible with the presence of the target but the process is not adaptive and no amplification of the preferred substrate can result; it is however sufficient for lead generation, i.e. the discovery of species presenting the activity sought for; in its second phase it amounts to the usual, static combinatorial chemistry approach where an actual, real library is screened by the target; the resulting library may be termed pre-equilibrated or postdynamic combinatorial library (pDCL).

In practice, it has been shown that in many cases the generation of a DCL must be carried out according to procedure b), due to the following reasons. The reactions leading to the formation of a DCL, i.e. the initial formation of bonds by reaction between functional groups on the building blocks (from which the library is generated) and the reaction of the compounds thus generated with further building blocks or with other compounds thus generated, until equilibrium is reached, often is too slow under conditions where the target is stable. The library is thus generated under non-physiological conditions (e.g. low pH) until equilibrium is reached, within an acceptable period of time. The reaction conditions are changed thereafter such that the target is stable. The target is then added, and the library is screened.

In Proc. Natl. Acad. Sci. USA 1997, 94, 2106 - 2110 L Huc and J.-M. Lehn disclose a method for the generation of a dynamic combinatorial library of imines from one set of aldehydes and one set of amines. The method is directed toward the synthesis of inhibitiors of the enzyme carbonic anhydrase by recognition-involved assembly. The synthesis of the above mentioned imines is carried out either in the presence of said enzyme (so-called adaptive combinatorial libraries) or said enzyme is added after equilibration (so-called pre-equilibrated dynamic or post-dynamic combinatorial libraries, pDCLs).

The PCT-application WO 01/64605 of the applicant discloses the *in situ* generation and screening of a dynamic combinatorial carbohydrate library against Concanavalin A. The plant lectin Concanavalin A belongs to the broad class of carbohydrate binding proteins. It is either present during library generation or is added after equilibration.

Hydrazines play an important role in the generation of DCLs, as they easily react with aldehydes and also ketones to hydrazones in a reversible reaction. This reaction is for example described in the above-mentioned publication by Huc and Lehn in Proc. Natl. Acad. Sci. USA 94, 2106 (1997).

The use of hydrazides NH₂NHC(O)- in DCC has been reported, see Sanders et al., J. Chem. Soc., Chem. Commun., 1761 (2000) and J. Chem. Soc., Chem. Commun. 1575, (1999). By the reaction with aldehydes, libraries containing macrocycles having acylhydrazone bridges are generated. The amplification (i.e. generation in higher amounts) of one or more components in the library can be attained by e.g. the addition of alkali metal ions. By the method according to Sanders et al., receptor molecules could be formed, for example for Li⁺-ions. Furthermore, the libraries are always generated in non-aqueous media, for example CHCl₃/MeOH.

Lehn et al. disclose (ChemBioChem. 2001, 438, and PCT-application WO 02/20435 of the applicant) a method for the generation of a DCL composed of interconverting acyl hydrazones which is screened towards inhibition of acetylcholinesterase. From a set of hydrazide and aldehyde building blocks a library was obtained. Of all possible acyl hydrazones formed, active compounds containing two terminal kationic recognition groups separated by an appropriate distance could be identified. The library was formed at a pH of 4.0, at which its formation occurs rapidly. The test of inhibitory activity was conducted at pH 7.2, at which the interconversion of the library constituents is essentially blocked.

There is a continuous search for reversible reactions which have never been used in DCC. The formation of imines, hydrazones and disulfides has been mentioned beforehand as examples for reactions which are appropriate for the generation and screening of a DCL. Due to the particular requirements of DCC, the number of appropriate reactions is limited. It would be desirable to have further chemical reactions appropriate for the use in DCC.

The object of the present invention is to find further reactions which lend themselves for the generation and the screening of a dynamic combinatorial library DCL. The reactions should take place in an aqueous medium and should not show the drawbacks of the methods as published to date. In particular, the reaction should enable the formation of fully dynamic libraries throughout the entire process comprising the generation and the screening of the library. The process should not require a pre-equilibration step. The static screening step, i.e. adding the target under non-reversible conditions, should not be necessary. It should be possible to carry out the process under physiological conditions (in aqueous media and a pH close to 7). No post-DCC step by which the compounds generated are converted into stable products should be necessary.

These objects are attained by the use of nitrones in the formation of a dynamic combinatorial library.

These objects are also obtained by a method of forming a dynamic combinatorial library of compounds which are potentially capable of binding to a target, which method comprises
i) selecting one first set of molecules which molecules each carry at least one hydroxylamine function;
ii) selecting at least one further set of molecules which molecules carry at least one carbonyl function;
iii) reacting the two sets of molecules with each other and at least temporarily in the presence of the target, under conditions where a reversible formation of nitrone functions between the hydroxylamine function and the carbonyl function on the molecules forming each of the sets occurs.

Preferably the formation of the library occurs under physiological conditions and is fully reversible throughout the entire formation of the library. In one embodiment of the present invention, one of the mentioned functional groups (hydroxyl amine and carbonyl) is present in a large excess with respect to the other functional group during the formation of the library. In a further embodiment, both functional groups are present in about equimolar amounts, or one functional group is present in only a slight excess.

In the context of the present invention, the term "large excess" means, for example, an at least 5-fold, at least 20-fold, or at least 50-fold.

The excess which is chosen depends on the individual target. A given target may require an excess of one functional group which is higher than for a different target.

The set of molecules which is present in low amounts (low concentration) determines the maximum concentration of compounds to be produced in the DCC reaction while the set of molecules at higher concentration ensures a minimum rate of equilibration. The first set of molecules is employed in a concentration which is close to the concentration of the target, in general in the low µM range, preferably about 1 to 100 µM. The other set of molecules is used in excess to maintain equilibrium rates. The concentration of the second set is usually in the higher µM to the low mM range, preferably 500 µM to 10 mM.

The method according to the present invention allows to generate libraries containing stable products. In the context of the present invention, "stable" means that the products can be isolated and do not decompose when the set of molecules present in excess is removed. In contrast, the formation of imines by the reaction between aldehydes and amines gives products which are only stable in the presence of a large excess of one set of starting products. In order to analyze the library (detect the products), the products formed have to be converted into stable species. In the case of imines, this is achieved by hydrogenating these into amines. The formation of DCL's by reactions which do not result in stable products in the sense of the definition given beforehand is not a part of the present invention.

The formation of nitrones is known as such. It comprises the reaction between a hydroxyl amine function and a carbonyl function and is depicted below in equation (I).

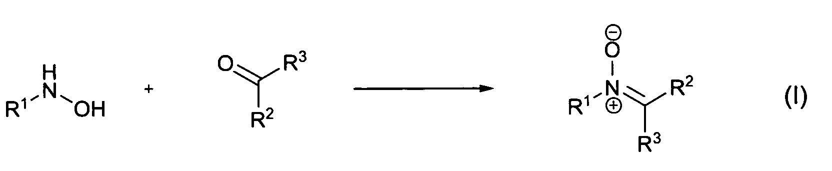

As a carbonyl function, all carbonyl functions known to the person skilled in the art can be used. Non-limiting examples include aldehydes, ketones, α-keto esters, β-keto esters, α-halo ketones, β-halo ketones, β―keto amides, α-alkoxy ketones, cyclic ketones, enones, α,β-unsaturated ketones, ynones, 1,2-diketones, 1,3-diketones, 1,4-diketones, α-oxyketones, β-oxy-ketones, α-amino-ketones, β-amino-ketones, α-keto sulfoxides, β-keto sulfoxides, α-nitro ketones, β-nitro ketones, α-keto sulfonic amides, β-keto sulfonic amides, α-keto sulfonats, β-keto sulfonats α-keto sulfonesters, β-keto sulfonesters. Particularly advantageous is the use of aldehydes, ketones and keto esters. Aldehydes are the most preferred compounds carrying a carbonyl function.

As the use of nitrones according to the present invention for the generation of a DCL is a general method and, in principle, not restricted neither to any particular hydroxyl amines nor to any particular carbonyl function carrying molecules, in the above equation (I), R¹, R² and R³ are thus not restricted and can be any appropriate organic rest; R³ can also be hydrogen. Organic rests include saturated and insaturated, linear and branched, cyclic and acyclic, substituted and unsubstituted aliphatic groups, which groups may contain hetero atoms as well as mono- and polynuclear, substituted and unsubstituted, optionally hetero atom containing aromatic groups. Suitable aliphatic groups may contain 1 to 100 carbon atoms, preferably 1 to 50 carbon atoms, in particular 1 to 20 carbon atoms. Suitable aromatic groups may contain 3 to 30, preferably 4 to 20 carbon atoms.

Suitable substituents are aliphatic groups and aromatic groups, halides, amino and organyl amino groups, nitro groups, carboxyl groups, ester groups, alcohol groups, thiol groups.

Hetero atoms include B, N, B, Si, N, P, O, S.

Various aspects of nitrone chemistry are for example disclosed in the following publication:
Tufariello, J.J. Nitrones in 1,3-dipolar Cycloaddition Chemistry, Padwa, A. (Ed.), John Wiley & sons, New York, 1984, Vol. 2, pp. 83-168.
The Chemistry of Carbon-Nitrogen Double Bond Patai, S. (Ed.), John Wiley & sons, New York, 1969.
The Chemistry of double bonded functional groups, Patai, S. (Ed.), John Wiley & sons, New York, 1977, Supplement A.

The excess values given above always refer to the functional groups. When, for example, the molecules in the first group of molecules carry the one functional group (carbonyl group or hydroxyl amine) and the molecules in the second group of molecules carry two of the other functional group, and the first functional group is to be used in excess, the first group of molecules has to be used in a 10-fold excess in order to reach a 5-fold excess of the first functional group relative to the second functional group.

As mentioned beforehand, the nitrone formation often does not require the use of a large excess of one of the functional groups employed. The nitrone formation and the exchange reaction are in general sufficiently fast to permit the equilibrium of the library to be attained within an acceptable time of several hours to a few days, e.g. 1 or 2 days, in the presence and the absence of the target, when employing equimolar amounts of functional groups or just a slight excess of one type of functional group. In case the reaction is not fast enough, a large excess of one type of functional groups can be used, in order to accelerate the formation of the equilibrium.

When in the context of the present invention the "generation" and the "screening" of a DCL are mentioned, one should be aware that these two steps can often not be separated in the formation of a DCL. For example, it is possible to mix the starting compounds with each other in the presence of the target which is then active in the entire process, meaning that the generation and the screening occur simultaneously. It is also possible, for example, to generate the library in the absence of the target, until equilibrium is reached, and then add the target. Although it can be said that the first step is the generation of the library and the second step the screening of it, this is not unambiguous as in the presence of the target the library is reformed or reequilibrated (i.e. the relative proportions of the components change, due to reversibility of the library-forming process), meaning that the generation of the DCL here also occurs, simultaneously.

The term "formation" of the library, as used in the context of the present invention, includes both steps, i.e. the generation and the screening of the library.

One advantage of employing nitrones in DCC is that a DCL can be generated in an aqueous medium, in particular an aqueous medium, which is close to or even at pH 7 and, hence, under physiological or almost physiological conditions, in the presence of the target, when the above-mentioned parameters are respected. The target - which in general is a biological target from the group of proteins - is stable under these conditions.

In the context of the present invention "close to pH 7" means a pH of from about 5 to 9. The value of choice depends on the stability of the specific target which may show its maximum activity at a pH of about 6 or of about 8. It is preferred to carry out the generation of the library at a pH of from about 6 to 8.

Preferably, the formation of the library is carried out in a buffered solution.

The dynamic process is fully operative throughout the entire process. The library can be formed first in the absence of the target, which is subsequently added to cause the amplification of the active compounds. As already mentioned above, the first step could be regarded as the generation of the DCL, whereas the second step could be regarded as the screening thereof. The library can also be generated directly in the presence of the target.

In any case, employing nitrones for the formation of a DCC according to the present invention allows the dynamic parameters to be fully operative throughout the entire formation of the library. Of course, the process may be carried out such the dynamic parameters are temporarily interrupted, as long as in the formation of the library an equilibrium can be reached. The dynamic process may also be interrupted after the library has attained equilibrium, for example in order to convert unstable into stable compounds before the analysis of the library.

Two different procedures can hence be employed. In one procedure, library generating and screening is performed in one single step; in the other procedure, these two steps are carried out separately. The particulars for the above-mentioned different procedures are the following:
a) adaptive, combinatorial libraries (one-step procedure): the generation of the library is conducted in a single step in the presence of the target, so that the library composition may adjust leading to selection and amplification of the preferred component(s); screening by the target occurs in parallel with the reversible generation of the library constituents; the molecules (building blocks) from which the library is formed and the resulting components can enter into contact with the target from the beginning:
b) pre-equilibrated dynamic combinatorial libraries (two-step procedure): the libraries are generated by reversible interconversion and equilibration in absence of the target (formation equilibrium), which is added in a second step (exchange equilibirium). The addition of the target thus occurs to the library which is fully active, i.e. which is still kept under equilibrium conditions. The initial equilibrium (formation equilibrium) is then shifted (in an exchange equilibrium) toward the species with the greatest affinity for the target. In the formation equilibrium, reversible bonds are initially formed and broken and reformed in a substitution reaction by the attack of a functional group. It is believed that due to the large excess of one type of functional groups relative to the other type of functional groups in the library, the formation equilibrium is fully (i.e. close to 100 %) on the product side. In the exchange equilibrium, the mixture of components initially formed then interconverts - by the action of the functional groups present in excess - to the mixture which is thermodynamically stable.

In a preferred embodiment of the present invention, the molecules in at least one set of molecules used for the generation of the library carry a functionality.

The term "functionality" used in the present invention designates a unit which can bind to the binding sites of the target, or interact with the target otherwise than by binding to it, for example by steric interactions.

"Functionality" means any polar, nonpolar, hydrophilic or lipophilic, or charged unit or subunit or electron donor or electron acceptor group. "Functionality" on the one hand includes simple functionalities like amino and imino groups and derivatives thereof, hydroxy and mercapto groups and derivatives thereof, oxo and thioxo groups, formyl and thioformyl groups, aryl groups, substituted aryl groups, phenyl groups, substituted phenyl groups, pyridyl groups and derivatives thereof, carboxy groups and carboxylato groups and derivatives therof, alkyloxycarbonyl groups, (di)thiocarboxy groups and derivatives thereof, (di)thiocarboxylato groups, carbamoyl groups and derivatives thereof, sulfo, sulfino and sulfeno groups and derivatives thereof, alkyloxysulfonyl, alkyloxysulfinyl and alkyloxysulfenyl groups, sulfamoyl, sulfinamoyl and sulfenamoyl groups and derivatives thereof, cyano and (iso)(thio)cyanato groups, hydroperoxy groups, nitroso groups, hydroxyamino groups, hydrazino groups, -NR¹R², -⁺NHR¹R² and -⁺NR¹R²R³ groups, wherein R¹, R², and R³ are identical or different and represent alkyl, cycloalkyl, alkylcycloalkyl, aryl, alkylaryl with 1 to 40 C atoms, -⁺OR¹R² groups wherein R¹ and R² are identical or different and represent alkyl, cycloalkyl, alkylcycloalkyl, aryl, alkylaryl with 1 to 40 C atoms, hydrazide groups and any other suitable groups known to a person skilled in the art.

On the other hand, "functionality" also includes more complex components, and nonlimiting examples include heterocycles carrying one or more heteroatoms in the ring selected from the group consisting of N, O and S, amino acids and oligo- and polypeptides, sugars (preferably hexoses and pentoses), sugar derivatives (like peracetylated sugars) and oligomers and polymers thereof, and nucleic acids and derivatives thereof.

The functionalities can thus be linked to a molecule, i.e. a molecule carrying the functionality (functionalities) is formed; the functionality can also itself be the molecule (and thus form the molecule carrying the functionality). An example of the latter case is a sugar molecule.

A molecule belonging to one set of molecules used for the generation of the library carries at least one "functional group" which term designates a group capable of reacting with another functional group, under the formation of a reversible chemical bond. The molecules carrying a functional group thus are the "building blocks" from which the (active) components are formed. In the present invention, the functional groups are restricted to carbonyl functions and hydroxyl amine functions.

When the molecules carrying a functional group (i.e. a carbonyl function or a hydroxyl amine function) combine with each other, "components" are formed. These components preferably already present the "ligands" which bind to the target. As according to the present invention the library should be fully reversible throughout the entire process of its formation (in the absence and in the presence of the target), no reaction should take place to transform the components to the ligands like is necessary, for example, in the reduction of generated imines to amines.

Component (ligand) means a molecule with a molecular weight typically not greater than 1500, preferably not greater than 1000, advantageously not greater than 500, which possesses an affinity for a target, i.e., that is able to interact with the target by forming one or a plurality of weak bonds such as hydrogen bonds, hydrophobic interactions, charge-charge interactions, Van der Waals interactions, donor-acceptor interactions, charge-transfer interactions, metal ion bindings, etc.

"Target" means a biological or synthetical macromolecule with a molecular weight typically greater than 5000. Biological macromolecules may be proteins including lipoproteins, glycoproteins and analogues of proteins, wherein either the peptide bond -CO-NH- is replaced by an analogous bond, possibly reversible such as an imine, ester, sulfonamide, sulfone, sulfoxide, phosphate, phosphonate, phosphonamide, guanidine, urea, thiourea, or imide bond, or wherein the aminoacids are replaced by synthetical derivatives thereof. The natural proteins may have differing functions, they may act namely as enzymes, as receptors or as antibodies. Receptors may be membrane receptors, hormone receptors, or signal transducers.

If the target is an enzyme, the ligand which is sought to be obtained may act as a substrate, an inhibitor or an activator for said enzyme.

If the target is a receptor, the ligand which is sought to be obtained may act as a natural or artificial ligand, an agonist or an antagonist for said receptor.

If the target is an antibody, the ligand which is sought to be obtained may act as an antigen for said antibody.

In general, all kinds of molecules, the one of which can act as a ligand and the other one as a target, are suitable to be used in the method according to the present invention.

The formation of a DCL employing nitrones comprises the use of scaffolds to which one or more aldehyde and/or hydroxyl amine functional group can be bound. Preferably, only one type of the functional groups mentioned beforehand is bound to the scaffold.

In case a scaffold is employed, one set of molecules employed in the formation of the library is found by the scaffold.

The scaffold can be any molecule which carries at least two functional groups, preferably at least three functional groups, and which has a structure which can potentially form the basis of an active component for a given target. In order to transform the scaffold into a component, the functionalities have to be attached to the scaffold. In a DCC-screening process, this occurs via the formation of reversible bonds by the interaction of functional groups on the molecule carrying the functional group and the scaffold. The scaffold can itself already have a certain activity with respect to the target, in general a biological activity. The scaffold can also show no activity. However, in any case, it is only after the attachment of functionalities to the scaffold that a lead compound, having a desired activity, is generated.

In one embodiment of the present invention, the scaffold is selected according to the requirements given by the binding site of the target. These requirements can be with respect to a certain size, three-dimensional structure and/or flexibility of the scaffold. It is mandatory that the scaffold can be easily controlled and operated under mild conditions. Furthermore, the compatibility with the bonding of the functionality to the target is an important feature of an appropriate scaffold for a given target. By the right choice of a scaffold having an appropriate size, three-dimensional structure and/or flexibility, the compounds formed, respectively the functionalities on the compounds, will fit into the binding site of the target and interact with it. One method to establish the appropriate size of the scaffold is, for example, analyzing the size of the binding site by methods known in the art like, for example, by X-ray structural analysis. It is also possible, for example, to evaluate the size of a known ligand for a given binding target and mimic this size.

The scaffold is a small organic or inorganic molecule. Organic molecules are preferred. In the context of the present invention, the term "small molecule" denotes a molecule which has a molecular weight in a region typical for organic molecules having a pharmacological activity and which are synthesized by chemical methods or are accessible by chemical methods. Preferably, the small molecules have a molecular weight of below about 500. The term "small molecule" does not refer to molecules of a high molecular with a pharmacological activity and which are typically synthesized by biochemical methods.

"Organic molecules" are molecules which contain at least one unit in the structure which is derived from the units -CH₃, -CH₂- or ≡CH wherein the hydrogen can partially or totally be replaced by substituents which can form a bond to the remaining carbon atom. Such substituents are known to the person skilled in the art and include, for example, halogen atoms, amino groups, ogygen atoms, hydroxy, alkoxy and aryloxy groups, thiol groups, organophosphine groups and organosilicon groups.

The organic molecule representing the scaffold can be of a cyclic or non-cyclic structure and can contain one or more heteroatoms, preferably from the group consisting of N, O, S, P, B, Si and halogens. The scaffold can also contain one or more double or triple bonds. The scaffold contains in its hydrocarbon chain at least one carbon atom, preferably at least three carbon atoms, in particular four to ten carbon atoms. The scaffold furthermore carries at least two functional groups, preferably two to six functional groups, more preferably two to four functional groups, in particular three or four functional groups which are selected from the group cited above in connection with the molecules carrying functional groups. The functional groups do not belong to the scaffold, as the term is understood in the context of the present invention. The scaffold can have one or more functionalities which are in accordance with the definition given above.

In both embodiments a) and b) for the formation of a library described above, the target can be used in a soluble form. The target can also be present in immobilized form, for example bound to a solid support. The target can also be used in its soluble form. After the generation of the library and the addition of the target (which is present either during the generation of the library or added afterwards, as laid out above) the reaction mixture containing the library is submitted to a work-up procedure which can be carried out in the following ways:
A) The target with the components bound to it is separated from the reaction mixture by methods known to the person skilled in the art. In the case of immobilized targets, the target is simply taken out of the mixture or filtered off. In the case of a soluble target, the target-component adduct can e.g. be removed by filtration techniques, in particular by ultrafiltration. After the separation of the target-component adduct the components are released. This can occur by destroying the target, for example by denaturation, in the case of a protein. It is sometimes also possible to release the component by changing the pH of the mixture. In particular, in the case where the target is a protein and protein-protein interactions are inhibited, a pH-change, for example by re-dissolving the target in a buffer solution, will release the bound components. After the release, the components are identified.
B) The target remains in the reaction mixture. It may not be necessary to release the bound components from the target, for example in cases when the target can promote formation of preferred species with a turnover. A more specific example for this are reactions in which the compounds of the generated library are transient species which have to be converted into stable species. One precise example are imines which have to be reduced to amines before analysis. The release of the components is however necessary in many cases. This is generally done with the methods known to the person skilled in the art, in particular with the methods which were described under A). In cases where the components are released, such release can occur simultaneously with the conversion of the transient species into stable species.

In order to identify the compounds which preferably bind to the target and which are hence preferably formed in the presence of the target, several possibilities exist. In one embodiment, the library formed in the presence of the target is analysed (i.e. the compounds formed are identified by analytical methods known to the person skilled in the art, preferably by mass spectrometry) and compared with a library formed under identical conditions, but in the absence of the target. In the context of the present invention, the library obtained after the "formation equilibrium" will thus in general be compared to the library obtained after the "exchange equilibrium". The compounds which preferably bind to the target are those which are formed in higher amounts in the presence of the target. This embodiment is preferred when the target is used in soluble form. The target is generally destroyed when the mixture is analysed.

It is also possible to isolate the target-ligand complex from the mixture, release the active compound (ligand) from the target and analyse the ligand. This is preferred in cases the target is used in immobilised form.

The present invention also comprises a pharmaceutically active compound synthesized according to the method according to the present invention, involving the formation of nitrone functions, and identifying the compound with the methods described above. The present invention furthermore comprises a pharmaceutical preparation which has been prepared from a pharmaceutically active compound obtained as described above which has been formulated, with a pharmaceutically acceptable carrier, into a pharmaceutical preparation. Optionally, the pharmaceutically active compound has been synthesized and isolated by methods known as such in amounts sufficient for the manufacture of a medicament.

The method of manufacturing and the pharmaceutical preparation are a part of the present invention.

The dynamic exchange (chemical reaction) between a nitrone group already formed and a hydroxyl amine function can be achieved by using an excess of hydroxyl amine with respect to the nitrone. As the nitrone is formed from carbonyl and hydroxyl amine groups, this means that an excess of hydroxyl amine groups is used relative to aldehyde groups. It is also possible to use an excess of carbonyl groups with respect to hydroxyl amine groups and the nitrone groups formed, respectively, to achieve an exchange between nitrone groups and carbonyl groups.

The present invention is now illustrated in the following examples.

### EXAMPLES

### Example 1 Reversible formation from building blocks

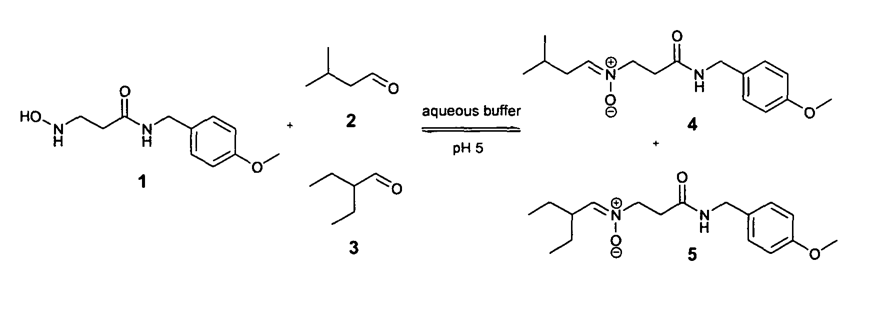

To a 31 µM solution of hydroxylamine 1 in ammonium acetate buffer (10 mM, pH 5, 4 ml) was added aldehyde **2** (2 µl of 1M solution in DMSO). After incubation for 24 h the reaction mixture was splitted in half. First mixture was analysed by HPLC/MS, to second mixture was added aldehyde **3** (1µl of 1M solution in DMSO). After additional incubation for 5h and 14h both samples were analysed by HPLCMS and results compared. MS data were acquired in full scan mode and scanned for protonated molecular ions [M+H]⁺ of potential nitrones in extracted ion chromatograms (EICs). In first mixture nitrone **4** could be detected LRMS (ESI+) m/z 293 ([M+H]⁺). In second mixture a distribution of both possible nitrones **4** and **5** could be detected. LRMS (ESI+) m/z 293 ([M+H]⁺); m/z 307 ([M+H]⁺)

### Example 2 Reversible exchange between nitrone and aldehydes

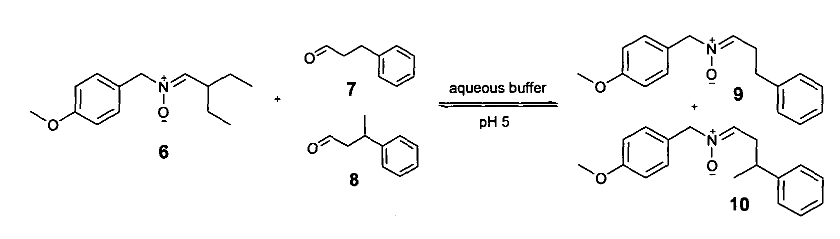

Nitrone **6** was synthesized following literature procedure (Dondoni, A. Synthesis of N-Benzyl Nitrones, *Synthetic Communication,* **1994,** 24 (18)) by condensation of 2-ethylbutyraldehyde (**3**) with *N*-(4-Methoxy-benzyl)-hydroxylamine. To a solution of crude aldehyde in chloroform was added the hydroxylamine followed by a large excess of magnesium sulphate. The reaction mixture was stirred at room temperature for 2.5 h and filtered. The filtrate was evaporated to yield a colourless solid. Identity and purity of crude nitrone was checked by ¹H-, ¹³C-NMR and HPLC/MS. ¹H-NMR (CDCl₃) δ 7.25 (d, 2H), 6.85 (d, 2H), 6.35 (d, 1H), 4.75 (s, 2H), 3.72 (s, 3H), 2.88 (m, 1H), 1.35 (m, 4H), 0.76 (t, 6H); ¹³C-NMR (CDCl₃) δ 160.4, 143.3, 131,1, 125.6, 114.6, 69.3, 55.7, 39.3, 24.8, 11.9; LRMS (ESI+) m/z 236 ([M+H]⁺). For DCC related experiments a 62 mM stock solution was prepared by dissolving 11 mg nitrone **6** in 740 µl DMSO.

The reversible exchange between synthesized nitrone and a mixture of aldehydes was tested by following experiment. To a 31 µM solution of nitrone **6** in ammonium acetate buffer (10 mM, pH 5, 2 ml) was added aldehyde **7** and **8** (1 µl of 1M solution in DMSO each). After incubation for 24 h the reaction mixture was analysed by HPLC/MS. MS data were acquired in full scan mode and scanned for protonated molecular ions [M+H]⁺ of potential nitrones in extracted ion chromatograms (EICs). A distribution of all possible nitrones could be detected. LRMS (ESI+) m/z 236 ([M+H]⁺); m/z 284 ([M+H]⁺); m/z 270 ([M+H]⁺).

### Example 3 Reversible exchange between nitrone and hydroxyl amines

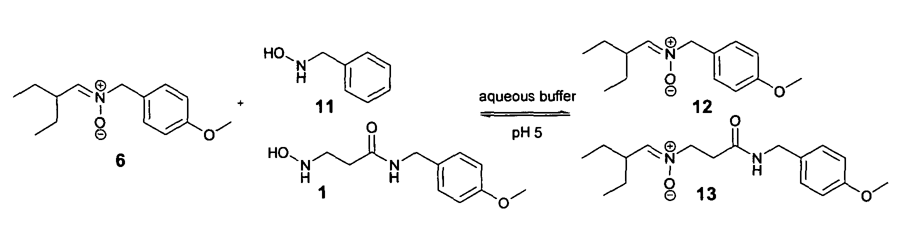

The reversible exchange between synthesized nitrone and a mixture of hydroxyl amines was tested by the following experiment. To a 31 µM solution of nitrone **6** in ammonium acetate buffer (10 mM, pH 5, 2 ml) was added hydroxyl amine **1** and **11** (4 µl of 62 mM solution in DMSO each). After incubation for 24 h the reaction mixture was analysed by HPLC/MS. MS data were acquired in full scan mode and scanned for all potential nitrones by generation of extracted ion chromatograms (EICs). A distribution of all possible nitrones could be detected. LRMS (ESI+) m/z 236 ([M+H]⁺); m/z 206 ([M+H]⁺); m/z 307 ([M+H]⁺).

Identity of new nitrone products was additional check by MS/MS experiments.

**Example 4** Scrambling of synthesized nitrones

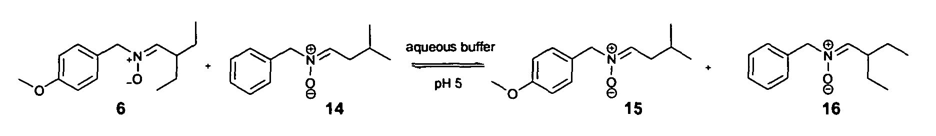

Nitrone **14** was synthesized according to literature procedure by condensation of 3-Methylbutyraldehyde with *N*-Benzyl-hydroxylamine in chloroform (see example 2). Identity and purity of synthesized nitrone was checked by ¹H-, ¹³C-NMR and HPLC/MS. LRMS (ESI+) m/z 192 ([M+H]⁺). For DCC related experiments a 62 mM stock solution was prepared by dissolving 13.7 mg nitrone **14** in 1.15 ml DMSO.

To ammonium acetate buffer (10 mM, pH 5, 998 µl) was added nitrone **6** and **14** (1µl of 62 mM solution in DMSO each). After Incubation for 12 h the reaction mixture was analyzed by HPLC/MS. MS data were acquired in full scan mode and scanned for protonated molecular ions [M+H]⁺ of potential products in extracted ion chromatograms (EICs). A distribution of all possible nitrones could be detected. LRMS (ESI+) m/z 192 ([M+H]⁺); m/z 222 ([M+H]⁺); m/z 206 ([M+H]⁺); m/z 236 ([M+H]⁺).

### Example 5 Formation of mixtures of nitrones from hydroxylamine and mixtures of building blocks

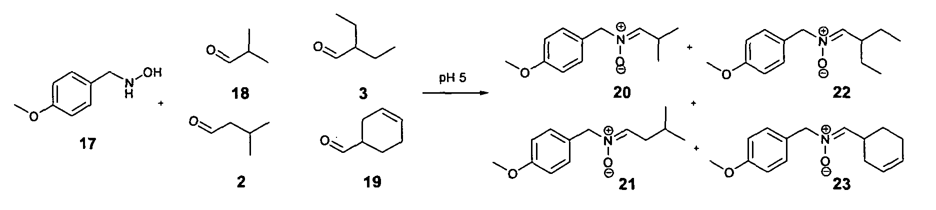

To a 20, 40, 60 µM solution of hydroxylamine **17** in ammonium acetate buffer (10 mM, pH 5, 1 ml) was added a mixture of five aldehydes (1 µl of 200/100/50/20 mM solution in DMSO each). After incubation the reaction mixture was analysed by HPLC/MS. MS data were acquired in full scan mode and scanned for protonated molecular ions [M+H]⁺ of potential products in extracted ion chromatograms (EICs). A distribution of possible nitrones could be detected. LRMS (ESI+) m/z 236 ([M+H]⁺); m/z 208 ([M+H]⁺); m/z 222 ([M+H]⁺); m/z 246 ([M+H]⁺)

## Claims

1. A method of forming a dynamic combinatorial library of compounds which are potentially capable of binding to a target, which method comprises
i) selecting one first set of molecules which molecules each carry at least one hydroxylamine function;
ii) selecting at least one further set of molecules which molecules carry at least one carbonyl function;
iii) reacting the two sets of molecules with each other and at least temporarily in the presence of the target, under conditions where a reversible formation of nitrone functions between the hydroxylamine function and the carbonyl function on the molecules forming each of the sets occurs.

2. The method according to claim 1, wherein the formation of the library occurs under physiological conditions.

3. The method according to claim 1 or 2, wherein the library is fully reversible throughout its entire formation.

4. The method according to any of claims 1 to 3, wherein one of the functional groups is present in a large excess with respect to the other functional group during its formation.

5. The method according to claim 4, wherein the excess is at least 5-fold.

6. The method according to claim 4, wherein the excess is at least 20-fold.

7. The method according to claim 4, wherein the excess is at least 50-fold.

8. The method according to any of claims 1 to 3, wherein both functional groups are present in about equimolar amounts, or one functional group is present in only a slight excess.

9. The method according to any of claims 1 to 8, wherein the target is employed in an amount in the µM range.

10. The method according to any of claims 1 to 9, wherein one set of molecules is employed in an amount close to that of the target.

11. The method according to any of claims 1 to 10, wherein the formation of the library is carried out in an aqueous medium.

12. The method according to any of claims 1 to 11, wherein the formation of the library is carried out at a pH of from about 5 to 9, preferably of from about 6 to 8.

13. The method according to any of claims 1 to 12, wherein the target is present throughout the entire process of the formation of the library.

14. The method according to any of claims 1 to 12, wherein the target is not present in the entire process of the formation of the library.

15. The method according to claim 14, wherein the libraries are generated by reversible interconversion and equilibration in absence of the target, which is added in a second step when the library is still kept under equilibrium conditions and which is fully active.

16. The method according to any of the claims 1 to 15, wherein the molecules carrying a carbonyl function are from the group consisting of aldehydes, ketones, α-keto esters, β-keto esters, α-halo ketones, β-halo ketones, β―keto amides, α-alkoxy ketones, cyclic ketones, enones, α,β-unsaturated ketones, ynones, 1,2-diketones, 1,3-diketones, 1,4-diketones, α-oxy-ketones, β-oxy-ketones, α-amino-ketones, β-amino-ketones, α-keto sulfoxides, β-keto sulfoxides, α-nitro ketones, β-nitro ketones, α-keto sulfonic amides, β-keto sulfonic amides, α-keto sulfonats, β-keto sulfonats α-keto sulfonesters, β-keto sulfonesters

17. The method according to any of the claims 1 to 16, wherein a scaffold is used.

18. The use of nitrones in the formation of a dynamic combinatorial library.

19. A dynamic combinatorial library which is obtained according to the method of any of claims 1 to 17.

20. A method of synthesizing and identifying a pharmaceutically active compound, which method comprises forming a library according to the method of any of claims 1 to 17, analysing the library by methods known as such and identifying the compound or compounds which preferably bind to the target.

21. The method according to claim 20, wherein the compound(s) binding preferably to the target is/are identified by comparing the amount of one or more compounds formed in the absence of the target with the respective amount formed in the presence of the target.

22. A pharmaceutically active compound synthesized and identified according to claim 20 or 21.

23. A method for the manufacture of a pharmaceutical preparation, comprising synthesizing and identifying a pharmaceutically active compound according to claim 20 or 21, optionally synthesizing and isolating the compound in amounts necessary for a medicament, and formulating the active compound and a pharmaceutically acceptable carrier into a pharmaceutical preparation.

24. A pharmaceutical preparation obtained according to the method of claim 23.
